Europäisches Patentamt

European Patent Office

Office européen des brevets

(11) Publication number: **0 414 404 A2**

# EUROPEAN PATENT APPLICATION

(21) Application number: **90308627.0**

(51) Int. Cl.5: **C12N 1/04**

(22) Date of filing: **06.08.90**

(30) Priority: **09.08.89 US 391748**

(43) Date of publication of application:
**27.02.91 Bulletin 91/09**

(84) Designated Contracting States:
**AT BE CH DE DK ES FR GB GR IT LI LU NL SE**

(71) Applicant: **Mycogen Corporation**
**5451 Oberlin Drive**
**San Diego, CA 92121(US)**

(72) Inventor: **Barnes, Andrew C.**
**13306 Landfair Road**
**San Diego, California 92130(US)**
Inventor: **Hannoun, Betty J.M.**
**7008 Weller Street**
**San Diego, California 92122(US)**
Inventor: **Nette, Kathryn M.**
**10262 Pinecastle Street**
**San Diego, California 92131(US)**
Inventor: **Gibb, Gregory D.**
**2709 Peachwood Trail**
**St. Louis, Missouri 63129(US)**
Inventor: **Hori, Hidetaka**
**Daigiri, 1108-2**
**Fujisawa, Kanagawa, 251(JP)**

(74) Representative: **Perry, Robert Edward et al**
**GILL JENNINGS & EVERY 53-64 Chancery**
**Lane**
**London WC2A 1HN(GB)**

(54) Process for encapsulation of biologicals.

(57) Disclosed is a process for encapsulation of biologicals. Specifically, microbes which have produced useful proteins, which are maintained intracellularly, are stabilized by treatment of the intact microbe with organic acids, inorganic acids, or oxidixing agents.

EP 0 414 404 A2

## PROCESS FOR ENCAPSULATION OF BIOLOGICALS

### Background of the Invention

The extraordinary increase in agricultural productivity has been a result of many factors, including significantly better understanding of the methods involved with agriculture, improved equipment, availability of fertilizers, and improved pesticides. The latter factor has not been without detrimental aspects, however, due to the negative effect on the environment. There is, therefore, a substantial interest in developing effective and environmentally acceptable pesticides.

Among ecologically acceptable pesticides are the protein toxins produced by various microorganisms, such as Bacillus thuringiensis . However, the use of B. thuringiensis lysate or spores as a pesticide has significant drawbacks. The lifetime of the pesticide is relatively short in the environment, requiring multiple applications to give adequate protection. Consequently, these pesticides are not economical in comparison to more traditional chemical products having long residual activities. Improvements in field longevity would greatly aid in expanding the application of biological, or protein toxin-based, pesticides.

West (1984) Soil Biol. Biochem. 16:357-360 reports the results of a study on the persistence of Bacillus thuringiensis ( B.t. ) toxin in soil. See also, West et al. (1984) J. of Invertebrate Pathology 43:150-155. U.S. Patent No. 4,265,880 describes embedding live insecticidal pathogens in a coacervate microbead. Japanese Patent No. 51-5047 describes physical-chemical methods for killing B.t. spores, while retaining toxicity. U.S. Patent No. 4,695,455 discloses procedures for cellular encapsulation of pesticides produced by expression of heterologous genes. U.S. Patent No. 4,695,462 discloses similar procedures applied to naturally-occurring pesticide-producing microorganisms. There is no dispute that the procedures disclosed in these two patents are very effective, with the resulting microbe-encapsulated pesticide having greater stability in the environment than the non-encapsulated pesticide. The invention disclosed herein results from further work on the chemical stabilization procedures disclosed in the patents.

### Brief Summary of the Invention

The subject invention concerns a process for stabilizing microbes contanining biologicals, e.g., pesticides, growth hormones, enzymes, nematicides insecticides, anti-infectives, antivirals, antifungals, and the like, by use of inorganic acids, organic acids, oxidizing agents, or any combination of these agents. These stabilizing agents are added, conveniently, to the fermentation culture medium prior to the harvesting of the microbe grown in the fermentation.

The microbe being grown, e.g., a pesticide-producing Pseudomonas or Bacillus thuringiensis microbe, can be a recombinant microbe having a heterologous gene which expresses the pesticide, or the microbe can be a non-recombinant microbe wherein a naturally-occurring gene in the microbe expresses the pesticide.

Examples of inorganic acids which can be used in the invention process are as follows: hydrochloric acid, nitric acid, sulfuric acid, periodic acid. phosphoric acid, sulfurous acid.

Examples of organic acids which can be used in the invention process are as follows: acetic acid, butyric acid, citric acid, formic acid, hexanoic acid. propionic acid, decanoic acid, carbolic acid, benzoic acid, lactic acid.

Examples of oxidizing agents which can be used in the invention process are as follows: hydrogen peroxide, acetic anhydride, benzoic anhydride.

Concentrations of the inorganic acids, organic acids, and oxidizing agents used in the invention process can range from about 0.1% to about 99%.

### Detailed Description of the Invention

Upon adding an inorganic acid, or organic acid, or oxidizing agent, or any combination of such reagents to a fermentation culture of a biological-producing microbe, prior to harvesting the microbe, microbe cells which are stabilized are obtained.

The chemical stabilizing agent can be mixed with the fermentation culture for about 0.5 to about 4 hours in order to complete the reaction of stabilizing the microbial cell. Shorter or longer periods of time can be used, depending on the progress of the stabilizing process.

It has been found, surprisingly and advantageously, that microbe cells stabilized by the subject invention process meet the stability requirements in the field of use for microbial pesticides. This is surprising because the disclosures of both U.S. Patent No. 4,695,455 and 4,695,462, the closest known prior art, which is discussed supra , used an acidic aqueous medium only in conjunction with the iodination process to stabilize the microbial cells. There is no suggestion in these patents, and hence no motivation, to use acids alone to stabilize the microbial cells.

Any microbe which produces a protein, polypeptide, amino acid, or other useful compound can be the starting material for the invention process. Examples of such microbially-produced products are growth hormones, enzymes, nematicides, insecticides, anti-infectives, antivirals, antifungals, and the like.

Thus, in accordance with an embodiment of the subject invention, improved pesticides are provided, having among their other advantages an extended residual life, by modifying pesticide-producing microorganisms hosting a natural (native) or heterologous gene capable of expression in the host, where expression of the gene results, directly or indirectly, in the production of a pesticide. The pesticide product retains the toxicity of the toxin.

A wide variety of pesticides can be produced which will be characterized by being capable of being produced intracellularly, particularly in a unicellular microorganism host, such as prokaryotes, e.g., bacteria; or eukaryotes, e.g., fungi, exemplified by yeast and filamentous fungi, such as Neurospora and Aspergillus ; or protists, such as amoebas, protozoa, algae, and the like.

The pesticide can be any toxin produced by a microbe. For example, it can be a polypeptide which has toxic activity toward a eukaryotic multicellular pest, such as insects, e.g., coleoptera, lepidoptera, diptera, hemiptera, dermaptera, and orthoptera; or arachnids; gastropods; or worms, such as nematodes and platyhelminths. Various susceptible insects include beetles, moths, flies, grasshoppers, lice, and earwigs.

The pesticide which is produced in the host cell may be a polypeptide produced in active form or a precursor or proform which requires further processing for toxin activity, e.g., by the pest, as with the crystal toxin of B. thuringiensis var. kurstaki . Thus, the gene may encode an enzyme which modifies a metabolite to produce a pesticidal composition.

Among naturally-occurring toxins are the polypeptide crystal toxins of B. thuringiensis var. kurstaki , active against lepidoptera; B thuringiensis var. israelensis , active against mosquitoes; B. thuringiensis var. san diego , active against coleoptera; B. thuringiensis var. aizawai , active against spodoptera; and B. sphaericus , active against mosquito larvae. Other toxins include those of entomopathogenic fungi, such as beauverin of Beauveria bassiana and destruxins of Metarhizium spp.; or the broad spectrum insecticidal compounds, such as the avermectins of Streptomyces avermitilus . Cultures exemplifying the above are as follows:

Bacillus thuringiensis var. kurstaki HD-1--NRRL B-3792; disclosed in U.S. Patent 4,448,885
Bacillus thuringiensis var. israelensis --ATCC 35646
Bacillus thuringiensis var. san diego --NRRL B-15939
Bacillus thuringiensis var. tenebrionis --DSM 2803 (German Collection of Microorganisms)

The following B. thuringiensis cultures are available from the United States Department of Agriculture (USDA) at Brownsville, Texas. Requests should be made to Joe Garcia, USDA, ARS, Cotton Insects Research Unit, P.O. Box 1033, Brownsville, Texas 78520 USA.

B. thuringiensis HD2
B. thuringiensis var. finitimus HD3
B. thuringiensis var. alesti HD4
B. thuringiensis var. kurstaki HD73
B. thuringiensis var. sotto HD770
B. thuringiensis var. dendrolimus HD7
B. thuringiensis var. kenyae HD5
B. thuringiensis var. galleriae HD29
B. thuringiensis var. canadensis HD224
B. thuringiensis var. entomocidus HD9
B. thuringiensis var. subtoxicus HD109
B. thuringiensis var. aizawai HD11
B. thuringiensis var. morrisoni HD12
B. thuringiensis var. ostriniae HD501
B. thuringiensis var. tolworthi HD537

B. thuringiensis var. darmstadiensis HD146
B. thuringiensis var. toumanoffi HD201
B. thuringiensis var. kyushuensis HD541
B. thuringiensis var. thompsoni HD542
B. thuringiensis var. pakistani HD395
B. thuringiensis var. israelensis HD567
B. thuringiensis var. indiana HD521
B. thuringiensis var. dakota
B. thuringiensis var. tohokuensis HD866
B. thuringiensis var. kumanotoensis HD867
B. thuringiensis var. tochigiensis HD868
B. thuringiensis var. colmeri HD847
B. thuringiensis var. wuhanensis HD525
Bacillus cereus --ATCC 21281
Bacillus moritai --ATCC 21282
Bacillus popilliae --ATCC 14706
Bacillus lentimorbus --ATCC 14707
Bacillus sphaericus --ATCC 33203
Beauveria bassiana --ATCC 9835
Metarrhizium anisopliae --ATCC 24398
Metarrhizium flavoviride --ATCC 32969
Streptomyces avermitilus --ATCC 31267

The toxin need not be the same as a naturally-occurring toxin. Polypeptide toxins may be fragments of a naturally-occurring toxin; expression products of deletion, transversion or transition mutations, where two or fewer number percent of the amino acids may be changed; or a repetitive sequence capable of processing by the intended pest host. In addition, fusion products may be prepared where one, five, or more amino acids are provided at the N-terminus to provide, for example, reduced proteolytic degradation of the toxin(s). In some instances, a plurality of the same or different toxins may be encoded and expressed, where processing sites may be introduced between each toxin moiety in the polytoxin.

Illustrative host cells include either prokaryotes or eukaryotes, normally being limited to those cells which do not produce substances toxic to higher organisms, such as mammals. However, organisms which produce substances toxic to higher organisms could be used, where the toxin is unstable or the level of application is sufficiently low as to avoid any possibility of toxicity to a mammalian host. Prokaryotes and lower eukaryotes, such as fungi, will be of particular interest as hosts. Illustrative prokaryotes, both Gram-negative and Gram-positive, include Enterobacteriaceae, such as Escherichia , Erwinia , Shigella , Salmonella , and Proteus ; Bacillaceae; Rhizobiaceae, such as Rhizobium ; Spirillaceae, such as photobacterium, Zymomonas , Serratia , Aeromonas , Vibrio , Desulfovibrio , Spirillum ; Lactobacillaceae; Pseudomonadaceae, such as Pseudomonas and Acetobacter ; Azotobacteraceae and Nitrobacteraceae. Among eukaryotes are fungi, such as Phycomycetes and Ascomycetes, which includes yeast, such as Saccharomyces and Schizosaccharomyces ; and Basidiomycetes yeast, such as Rhodotorula , Aureobasidium , Sporobolomyces and the like.

Characteristics of particular interest in selecting a host cell for purposes of production include ease of introducing the heterologous gene into the host, availability of expression systems, efficiency of expression, stability of the pesticide in the host, and the presence of auxiliary genetic capabilities. Characteristics of interest for use as a pesticide microcapsule include protective qualities for the pesticide, such as thick cell walls, pigmentation, and intracellular packaging or formation of inclusion bodies; leaf affinity; lack of mammalian toxicity; attractiveness to pests for ingestion; ease of killing and fixing without damage to the toxin; and the like. Other considerations include ease of formulation and handling, economics, storage stability, and the like.

Host organisms of particular interest include yeasts, such as Rhodotorula sp., Aureobasidium sp., Saccharomyces sp., and Sporobolomyces sp.; phylloplane organisms such as Pseudomonas sp., Erwinia sp., and Flavobacterium sp.; or such other organisms as Escherichia , Lactobacillus sp., Bacillus sp., and the like. Specific organisms include Pseudomonas aeruginosa , Pseudomonas fluorescens , Saccharomyces cerevisiae , Bacillus thuringiensis , Escherichia coli , Bacillus subtilis , and the like.

The cell will usually be intact and be substantially in the proliferative form when stabilized (killed). The cells may be inhibited from proliferation as disclosed herein. The invention process does not deleteriously affect the properties of the pesticide, nor diminish the cellular capability in protecting the pesticide.

The cellular host containing the heterologous pesticidal gene may be grown in any convenient nutrient medium, where the DNA construct provides a selective advantage, providing for a selective medium so that substantially all or all of the cells retain the heterologous gene. These cells may then be harvested in accordance with conventional ways, and then treated with a stabilizing agent. Preferably, the cells are treated prior to harvesting.

The cells may be formulated in a variety of ways. They may be employed as wettable powders, granules, or dusts, by mixing with various inert materials, such as inorganic materials (phyllosilicates, carbonates, sulfates, phosphates, and the like) or botanical materials (powdered corncobs, rice hulls, walnut shells, and the like). The formulations may include spreader-sticker adjuvants, stabilizing agents, other pesticidal additives, or surfactants. Liquid formulations may be aqueous-based or non-aqueous and employed as foams, gels, suspensions, emulsifiable concentrates, or the like. The ingredients may include rheological agents, surfactants, emulsifiers, dispersants, or polymers.

The pesticidal concentration will vary widely depending upon the nature of the particular formulation, particularly whether it is a concentrate or to be used directly. The pesticide will be present in at least 1% by weight, and may be 100% by weight. The dry formulations will have from about 1-95% by weight of the pesticide, while the liquid formulations will generally be from about 1-60% by weight of the solids in the liquid phase. The formulations will generally have from about $10^2$ to about $10^{10}$ cells/mg. These formulations will be administered at about 2 oz. (liquid or dry) to 10 or more lb/ha.

The formulations can be applied to the environment of the pest(s), e.g., plants, soil, or water, by spraying, dusting, sprinkling, or the like.

In another embodiment of the subject invention, a wide variety of proteins can be produced which will be characterized by being capable of being produced intracellularly, particularly in a unicellular microorganism host, such as prokaryotes, e.g., bacteria; or eukaryotes, e.g., fungi, exemplified by yeast and filamentous fungi, such as Neurospora and Aspergillus ; or protists, such as amoebas, protozoa, algae, and the like.

The protein can be a nematicide produced by a Bacillus thuringiensis ( B.t. ) microorganism. In such a case the B.t. microbe can be treated in accord with the process disclosed herein to provide an economical and efficient delivery system to treat animals and humans afflicted with nematodes. The group of diseases described generally as helminthiasis is due to infection of an animal host with parasitic worms known as helminths. Helminthiasis is a prevalent and serious economic problem in domesticated animals such as swine, sheep, horses, cattle, goats, dogs, cats, and poultry. Among the helminths, the group of worms described as nematodes causes widespread and often serious infection in various species of animals. The most common genera of nematodes infecting the animals referred to above are Haemonchus , Trichostrongylus , Ostertagia , Nematodirus , Cooperia , Ascaris , Bunostomum , Oesophagostomum , Chabertia , Trichuris , Strongylus , Trichonema , Dictyocaulus , Capillaria , Heterakis , Toxocara , Ascaridia , Oxyuris , Ancylostoma , Uncinaria , Toxascaris , Caenorhabditis , and Parascaris . Certain of these, such as Nematodirus , Cooperia , and Oesophagostomum , attach primarily the intestinal tract, while others, such as Dictyocaulus are found in the lungs. Still other parasites may be located in other tissues and organs of the body.

Products encapsulatable by the process of the subject invention, advantageously, can be targeted to be released at a specific site in the animal or human host, or in the target pest. This "targeting of release" can be achieved by the nature of treatment of the microbial cell containing the desired product. For example, certain products, advantageously, are released in the animal digestive system. Examples of such products are growth hormones, therapeutic agents, intestinal parasite pesticides, and the like. Other products, advantageously, will pass through the animal or human host substantially intact and maintain the encapsulated protein substantially in the active form in the excrement or fecal matter. This targeted release would, for example, limit the proliferation of flies in waste matter.

Microbes suitable for treatment in the subject invention can be any microbe which itself would not be toxic to the animal or human host when administered in the treated form. It should be recognized that the treatment of the microbe will render the microbe non-proliferative. At any rate, a large number of microbes are known which are not toxic per se to animals and humans.

Illustrative host cells may include either prokaryotes or eukaryotes, normally being limited to those cells which do not produce substances toxic to animals. However, organisms which produce substances toxic to higher organisms could be used, where the toxin is unstable or the level of application sufficiently low as to avoid any possibility of toxicity to an animal host.

When the treated microbe, e.g., B.t. microbe, is administered as a component of the feed of animals, or dispersed or suspended in the drinking water, compositions are provided in which the encapsulated B.t. microbe is intimately dispersed in an inert carrier or diluent. By inert carrier is meant one that will not react with the antiparasitic agent and one that may be administered safely to animals. Preferably, a carrier for feed administration is one that is, or may be, an ingredient of the animal ration.

Suitable compositions include feed premixes or supplements in which the treated B.t. microbe is present in relative large amounts and which are suitable for direct feeding to the animal or for addition to the feed, either directly or after an intermediate dilution or blending step. Typical carriers or diluents suitable for such compositions include, for example, distillers' dried grains, corn meal, citrus meal, fermentation residues, ground oyster shells, wheat shorts, molasses solubles, corn cob meal, edible bean mill feed, soya grits, crushed limestone, and the like.

In addition to having anthelminthic activity within the digestive tract of animals, appropriately treated B.t. isolates will pass through the animals' digestive tract and thereby provide additional control of nematode larvae which hatch and multiply in the feces.

The protein which is produced in the host cell may be a polypeptide produced in active form or a precursor or proform which requires further processing for activity. Thus, the gene may encode an enzyme which modifies a metabolite to produce a protein composition.

When the microbial cell is treated, as described herein, the cells generally will have enhanced structural stability which will enhance resistance to premature breakdown in the animal or human host.

Following are examples which illustrate procedures, including the best mode, for practicing the invention. These examples should not be construed as limiting. All percentages are by weight and all solvent mixture proportions are by volume unless otherwise noted.

Example 1 - Culturing an isolate Prior to Treatment

A subculture of a Pseudomonas containing a heterologous gene product can be used to inoculate the following medium:

| Tryptone | 10.0 g/l |
| Yeast extract | 5.0 g/l |
| NaCl | 5.0 g/l |
| pH 7.0 | |

Flasks are incubated at 30°C on a rotary shaker at 200 rpm for 64 hours.

The above procedure can be readily scaled up to large fermentors by procedures well known in the art.

Example 2 - Stabilization of Microbial Cells by Treatment with Glacial Acetic Acid

Pseudomonas cells, described in Example 1, are treated with about 5% (final concentration) glacial acetic acid. Acid and broth are mixed for about 2 hours. The cells are then harvested by centrifugation and formulated for use.

Example 3

By substituting the glacial acetic acid of Example 2 with about 1.5% phosphoric acid, stabilized microbial cells are obtained.

Example 4

By substituting the glacial acetic acid of Example 2 with about 1.2% acetic acid following by about 0.6% phosphoric acid, stabilized microbial cells are obtained.

Example 5

By substituting the glacial acetic acid of Example 2 with about 2% acetic anhydride, stabilized microbial cells are obtained.

Example 6 - Treated *Bacillus thuringiensis* Isolates

Substantially intact cells of B.t. isolates are treated via the invention process. The treated cells can be utilized as a feed supplement with nematicidal properties. By varying the treatment conditions, the B.t. cells can be made to substantially break down in the intestine, or, if desired, pass through the animal substantially intact and be excreted to act as a nematicide in the feces.

Example 7 - Treated *Bacillus thuringiensis* var. *israelensis (B.t.i.)* Cells

Substantially intact cells of a B.t.i. microbe, of which many are publicly known and available, are treated via the invention process. The treated cells can be utilized as a feed supplement with dipteran activity, e.g., activity against flies. The treatment of the cells is such that a substantial fraction of the treated B.t.i. cells pass through the animal and are excreted in the feces to control flies attracted to the feces.

Likewise, appropriately treated B.t.i. cells also can be deployed on lakes, rivers, ponds, and other areas infested with mosquitoes and/or flies.

## Claims

1. A stabilized microbe comprising a desired intracellular compound produced by the microbe, while substantially intact, as stabilized by treatment with a compound selected from organic acids, inorganic acids and oxidising agents.

2. A microbe according to claim 1, wherein the inorganic acids are selected from hydrochloric, nitric, sulfuric, periodic, phosphoric and sulfurous acids, the organic acids are selected from acetic, butyric, citric, formic, hexanoic, propionic, decanoic, carbolic, benzoic and lactic acids, and the oxidising agents are selected from hydrogen peroxide, acetic anhydride and benzoic anhydride.

3. A microbe according to claim 1 or claim 2, which is a yeast or a prokaryote selected from Pseudomonas microbes and Bacillus thuringiensis .

4. A microbe according to claim 3, which is a nematicide- or insecticide-producing strain of Bacillus thuringiensis .

5. A microbe according to claim 1 or claim 2, which is a prokaryote selected from Enterobacteriaceae , Bacillaceae , Rhizobiaceae , Spirillaceae , Lactobacillaceae , Pseudomonadaceae , Azotobacteraceae and Nitrobacteraceae , or a eukaryote selected from Phycomycetes , Ascomycetes and Basidiomycetes .

6. A composition according to claim 1 or claim 5, which is a pigmented bacterium, yeasts or bacterium.

7. A microbe according to any preceding claim, wherein the intracellular compound is selected from growth hormones, enzymes, nematicides, insecticides, anti-infectives, anti-virals and anti-fungals.

8. A microbe according to claim 7, wherein the intracellular compound is a nematicide.

9. A microbe according to claim 7, wherein the intracellular compound is a pesticide.

10. A microbe according to any of claims 1 to 8, for therapeutic use.

11. Use of a microbe according to claim 8, for the manufacture of a medicament for use in treating an animal or human hosting a nematode infestation.

12. A feed supplement comprising a microbe according to any of claims 1 to 9.

13. A pesticidal composition comprising a microbe according to claim 9, and an inert carrier.